Europäisches Patentamt

European Patent Office

Office européen des brevets

(19)

(11) Publication number: **0 504 628 A2**

# EUROPEAN PATENT APPLICATION

(12)

(21) Application number: **92103204.1**

(22) Date of filing: **25.02.92**

(51) Int. Cl.⁵: **C07C 209/82**

(30) Priority: **18.03.91 US 670724**
**18.03.91 US 671031**
**18.03.91 US 671030**
**18.03.91 US 671033**
**18.03.91 US 670947**
**18.03.91 US 671032**
**18.03.91 US 670568**

(43) Date of publication of application:
**23.09.92 Bulletin 92/39**

(84) Designated Contracting States:
**BE DE ES FR GB IT**

(71) Applicant: **ETHYL CORPORATION**
**451 Florida Boulevard**
**Baton Rouge, Louisiana 70801(US)**

(72) Inventor: **Smith, Kim Renae**
**1950 Stafford Drive**
**Baton Rouge, Louisiana 70810(US)**
Inventor: **Borland, James Ellwood**
**12253 East Millburn**
**Baton Rouge, Louisiana 70815(US)**
Inventor: **Sauer, Joe Dean**
**15553 Waywood Avenue**
**Baton Rouge, Louisiana 70816(US)**

(74) Representative: **Sandmair, Kurt, Dr. Dr.**
**Patentanwälte Schwabe, Sandmair, Marx**
**Stuntzstrasse 16**
**W-8000 München 80(DE)**

(54) **Process for treating tertiary amines.**

(57) A tert-amine which contains an acid-activated color body precursor is treated to prevent it or a derivative thereof, e.g., an amine oxide, betaine, or quaternary ammonium compound, from turning pink when exposed to acidic conditions by contacting it at an effective temperature with a discoloration inhibitor which is (1) an inorganic sulfite selected from alkali metal sulfites and bisulfites, (2) an alkanal containing at least two carbons, (3) an acetylalkylcarbinol, (4) an amido compound which contains a -CO.NH-group, (5) ammonium carbonate, (6) a dialkylacetal, or (7) ascorbic acid. Ambient temperatures are satisfactory for the treatments with the other discoloration inhibitors, but temperatures above 100°C are required when the discoloration inhibitor is an inorganic sulfite.

EP 0 504 628 A2

## Field of Invention

This ivention relates to a process for treating a tert-amine to prevent pink coloration of the amine or a derivative thereof when exposed to acidic conditions.

## Background

As disclosed in U.S. Patent 4,347,381 (Tuvell), it is known that some tert-amines and derivatives thereof, e.g., amine oxides, betaines, and quaternary ammonium compounds, are subject to turning pink when exposed to acidic conditions. Neither the pink color body that is formed under acidic conditions nor its acid-activated precursor has yet been identified, and it is not known if the presence of the color body precursor as an impurity in some tert-amines is attributable to the raw materials and/or the techniques used in their syntheses. However, regardless of the primary reason for the discoloration, the pink coloration of the amines and their derivatives is undesirable because of its interference with dyes which are apt to be included in the formulations in which they are used.

A pink tert-amine or derivative thereof can have its color reduced in intensity or even removed by treating it with a bleaching agent, such as hydrogen peroxide, sodium sulfite, sodium bisulfite, sodium thiosulfate, or sodium hypochlorite, as in Tuvell; but it would be preferable to prevent the discoloration from occurring rather than to remove or reduce the discoloration later.

U.S. Patent 3,922,306 (Takaku et al.) teaches that this discoloration of amine salts can sometimes be prevented when the amines are reacted with 0.01-5% by weight of certain borohydrides. According to Takaku et al., the effectiveness of the borohydrides in this regard is unexpected and unique, since it cannot be attained by the use of other reducing agents, such as the sodium sulfite or bisulfite, hydrazine, or hypophosphorous acid of their comparative example. However, it would be desirable to find an alternative to the borohydrides as agents for preventing discoloration of the tert-amines and derivatives. As indicated by Tuvell, the borohydrides are too expensive to make their use commercially attractive.

## Summary of Invention

It has been found that a tert-amine which contains an acid-activated color body precursor can be treated to prevent it or a derivative thereof from turning pink when exposed to acidic conditions by contacting it at an effective temperature with a discoloration inhibitor which is (1) an inorganic sulfite selected from alkali metal sulfites and bisulfites, (2) an alkanal containing at least two carbons, (3) an acetylalkylcarbinol, (4) an amido compound which contains a -CO.NH- group, (5) ammonium carbonate, (6) a dialkylacetal, or (7) ascorbic acid.

## Detailed Description

As is known, the tert-amines which present the aforementioned discoloration problem are amines which contain at least one long-chain alkyl group, and any of these tert-amines can be treated in accordance with the present invention. However, because of their use in preparing derivatives having the greatest commercial interest, the tert-amines which are treated are preferably compounds corresponding to the formula RR'R''N in which R is an alkyl group containing 8-22 carbons; R' is a methyl, ethyl, or hydroxyethyl group; and R'' is independently selected from methyl, ethyl, hydroxyethyl, and alkyl groups containing 8-22 carbons.

Of the tert-amines in which R' and R'' are independently selected from methyl, ethyl, and hydroxyethyl, those which are of greatest interest are generally the tert-amines in which both R' and R'' are methyl; and it is usually also preferred for R' to be methyl in the tert-amines wherein R and R'' are independently selected from alkyl groups containing 8-22 carbons.

Exemplary of these tert-amines are N-octyldiethylamine, N-octyl-N-hydroxyethylmethylamine, N,N-didecylmethylamine, N-dodecyl-N-tetradecylhydroxyethylamine, N,N-ditetradecylmethylamine, N-tetradecyldimethylamine, N-hexadecyl-N-ethylmethylamine, N-octadecyl-N-eicosylmethylamine, N-docosyldimethylamine, and N-tetracosyldimethylamine.

The discoloration inhibitor with which the tert-amine is contacted may be any of the variety of compounds described above. However, as noted hereinafter, some such compounds are apt to be preferred. Thus, e.g.:

(A) When an inorganic sulfite is employed, it may be a sulfite or bisulfite of any alkali metal, i.e., lithium, sodium, potassium, rubidium, or cesium; but it is preferably sodium sulfite or bisulfite.

(B) An alkanal used in the practice of the invention may be any alkanal containing at least two carbons, and there does not appear to be any maximum to the number of carbons it may contain from the viewpoint of effectiveness. From the aspect of cost and availability, however, it is generally preferred that the alkanal be one containing 2-20 carbons, such as ethanal, propanal, α-methylpropanal, butanal, β-methylbutanal, pentanal, hexanal, heptanal, octanal, nonanal, decanal, undecanal, dodecanal, tridecanal, tetradecanal, hexadecanal, octadecanal, or eicosanal.

(C) An acetylalkylcarbinol that is utilized is preferably one in which the alkyl group contains 1-20 carbons, e.g., the acetylalkylcarbinols wherein the alkyl group is methyl, ethyl, propyl, isopropyl, butyl, hexyl, decyl, hexadecyl, or eicosyl; and acetylmethylcarbinol is usually most preferred.

(D) Utilizable amido compounds containing a -CO.NH- group include, e.g., amides derived from an alkanoic or benzoic acid, anilides or other N-monosubstituted derivatives of such amides, heterocyclic amides, urea, and alkyl carbamates. Exemplary of such compounds are formamide, acetamide, butyramide, palmitamide, stearamide, benzamide, N-methylformamide, N-ethylacetamide, acetanilide, benzanilide, 2-pyrrolidone, urea, and the alkyl carbamates in which the alkyl group is methyl, ethyl, propyl, isopropyl, butyl, hexyl, decyl, tetradecyl, or other alkyl group containing 1-20 carbons.

(E) The dialkylacetals which may be used are those which can be defined as a reaction product between an alkanol - usually an alkanol containing 1-20 carbons, such as methanol, ethanol, propanol, $\alpha$-methylpropanol, butanol, hexanol, decanol, tetradecanol, or eicosanol - and an aldehyde which also typically contains 1-20 carbons, e.g., methanal, ethanal, $\beta$-acetylethanal, propanal, $\alpha$-methylpropanal, butanal, $\beta$-methylbutanal, pentanal, hexanal, octanal, decanal, undecanal, dodecanal, tetradecanal, hexadecanal, octadecanal, nonadecanal, or eicosanal.

The amount of discoloration inhibitor employed is generally at least about 0.1%, based on the weight of the tert-amine, although lesser amounts are effective in at least reducing the discoloration that occurs under acidic conditions and thus can be usefully employed if desired.

Since treatment with about 0.1% by weight of the discoloration inhibitor is usually sufficient to prevent the tert-amine or a derivative thereof from turning pink when exposed to acidic conditions, it is seldom necessary to use a larger amount; but larger amounts can be utilized without deleterious effect. Thus, in most cases, the tert-amine is contacted with 0.05-10%, preferably 0.1-5%, most preferably 0.1-0.5% of the discoloration inhibitor, based on the weight of the tert-amine.

The treatment of the tert-amine with the discoloration inhibitor is believed to result in a reaction between the discoloration inhibitor and the acid-activated color body precursor and a consequent prevention of the formation of a color body when the tert-amine or a derivative thereof is exposed to acidic conditions. This treatment is accomplished by contacting the tert-amine and discoloration inhibitor at an effective temperature in any suitable way, e.g., by dissolving or slurrying the inhibitor in the tert-amine, and then removing the inhibitor, e.g., by distillation or filtration, or - in some cases, e.g., when an inorganic sulfite is used - by passing the tert-amine over a bed of the inhibitor.

When the discoloration inhibitor is an inorganic sulfite, the effective temperature for the tert-amine treatment is a temperature which is above 100°C but below the decomposition temperature of the amine, generally a temperature in the range of 100-300°C, preferably 100-200°C; and the most suitable time for maintaining contact between the tert-amine and the sulfite is usually at least one hour but not more than four hours.

When the discoloration inhibitor is one of the utilizable compounds other than an inorganic sulfite, the effectiveness of the treatment in preventing later discoloration of the tert-amine is probably at least virtually instantaneous even at room temperature; but it is generally desirable to maintain contact between the tert-amine and inhibitor for a short time, e.g., 1-30 minutes, to insure that effectiveness. Also, an elevated temperature could be used for the treatment without deleterious effect. However, it is preferred to conduct the treatment at ambient temperatures.

The invention is advantageous as an economical means of treating tert-amines so that neither they nor their derivatives, e.g., amine oxides, betaines, and quaternary ammonium compounds, will turn pink when exposed to acidic conditions.

The following examples are given to illustrate the invention and are not intended as a limitation thereof. Unless otherwise specified, quantities mentioned in the examples are quantities by weight.

EXAMPLE A (COMPARATIVE)

An aliquot of a distilled N-tetradecyldimethylamine was acidified with HCl. Below a pH of 6-7, it developed an intense pink color.

EXAMPLE 1

A 1% slurry of sodium sulfite in another aliquot of the distilled amine of Example A was prepared and heated to 180°C. After the slurry was kept at 180°C for four hours, the sulfite was removed by filtration. When treated in this manner and then acidified with HCl as in Example A, the amine remained colorless.

EXAMPLE 2

A 1% solution of decanal in another aliquot of the distilled amine of Example A was shaken at room temperature for 15 minutes and then distilled to remove the aldehyde. When treated in this manner and then acidified with HCl as in Example A, the amine remained colorless.

EXAMPLES 3-7

Five experiments were performed by repeating Example 2 except for replacing the decanal with acetylmethylcarbinol, formamide, 2-pyrrolidone, methyl carbamate, and acetylacetaldehyde dimethylacetal, respectively. In each case the treated amine remained colorless when acidified.

EXAMPLE 8

A 1% slurry of urea in another aliquot of the distilled amine of Example A was shaken at room temperature for 15 minutes and then filtered to remove the urea. When treated in this manner and then acidified with HCl as in Example A, the amine remained colorless.

EXAMPLE 9

A 1% slurry of ammonium carbonate in another aliquot of the distilled amine of Example A was shaken at room temperature for 15 minutes and then distilled to remove the carbonate. When treated in this manner and then acidified with HCl as in Example A, the amine remained colorless.

EXAMPLE 10

A 1% slurry of ascorbic acid in another liquot of the distilled amine of Example A was shaken at room temperature for 15 minutes and then distilled to separate the amine from the amine/ascorbic acid salt. When treated in this manner and then acidified with HCl as in Example A, the amine remained colorless.

**Claims**

1. A process for treating a tert-amine that contains an acid-activated color body precursor so as to prevent pink coloration of the tert-amine or a derivative thereof when exposed to acidic conditions, which process comprises contacting the tert-amine at an effective temperature with a discoloration inhibitor which is (a) an inorganic sulfite selected from alkali metal sulfites and bisulfites, (b) an alkanal containing at least two carbons, (c) an acetylalkylcarbinol, (d) an amido compound which contains a -CO.NH- group, (e) ammonium carbonate, (f) a dialkylacetal, or (g) ascorbic acid.

2. The process of claim 1 wherein the discoloration inhibitor is an inorganic sulfite, which is contacted with the tert-amine at a temperature above 100°C for at least one hour.

3. The process of claim 2 wherein the temperature is in the range of 100-300°C.

4. The process of claim 2 wherein the inorganic sulfite is sodium sulfite.

5. The process of claim 1 wherein the discoloration inhibitor is an alkanal containing 2-20 carbons.

6. The process of claim 5 wherein the alkanal is decanal.

7. The process of claim 1 wherein the discoloration inhibitor is an acetylalkylcarbinol in which the alkyl group contains 1-20 carbons.

8. The process of claim 7 wherein the acetylalkylcarbinol is acetylmethylcarbinol.

9. The process of claim 1 wherein the discoloration inhibitor is an amido compound selected from the group consisting of amides derived from alkanoic and benzoic acids and N-monosubstituted derivatives thereof, heterocyclic amides, urea, and alkyl carbamates.

10. The process of claim 9 wherein the amido compound is formamide, 2-pyrrolidone, urea, or methyl carbamate.

11. The process of claim 1 wherein the discoloration inhibitor is ammonium carbonate.

12. The process of claim 1 wherein the discoloration inhibitor is a dialkylacetal of an alkanol containing 1-20 carbons and an aldehyde containing 1-20 carbons.

13. The process of claim 12 wherein the acetal is acetylacetaldehyde dimethylacetal.

14. The process of claim 1 wherein the discoloration inhibitor is ascorbic acid.

15. The process of any of claims 5-14 wherein the tert-amine is maintained in contact with the discoloration inhibitor for 1-30 minutes at ambient temperature.

16. The process of any of the preceding claims wherein the amount of discoloration inhibitor is at least about 0.1%, based on the weight of the tert-amine.

17. The process of any of the preceding claims wherein the tert-amine is a compound corresponding to the formula RR'R''N in which R is an alkyl group containing 8-22 carbons; R' is a methyl, ethyl, or hydroxyethyl group; and R'' is independently selected from methyl, ethyl, hydroxyethyl, and alkyl groups containing 8-22 carbons.

18. The process of claim 17 wherein R' and R'' are methyl.

19. The process of claim 17 wherein R' is methyl and R'' is an alkyl group containing 8-22 carbons.